(19) Europäisches Patentamt
European Patent Office
Office européen des brevets

(11) Numéro de publication: **0 240 423**
**A2**

(12) **DEMANDE DE BREVET EUROPEEN**

(21) Numéro de dépôt: **87400693.5**

(22) Date de dépôt: **27.03.87**

(51) Int. Cl.[4]: **C 12 P 13/00**
C 12 P 1/00

(30) Priorité: **04.04.86 FR 8604832**

(43) Date de publication de la demande:
**07.10.87 Bulletin 87/41**

(84) Etats contractants désignés:
**AT BE CH DE ES GB GR IT LI LU NL SE**

(71) Demandeur: **SOCIETE NATIONALE ELF AQUITAINE**
**Société anonyme dite**
**Tour Elf 2, Place de la Coupole La Défense 6**
**F-92400 Courbevoie (FR)**

(72) Inventeur: **Souppe, Jérôme**
**3 rue de Babsalle**
**F-64000 Pau (FR)**

**Haurat, Gisèle**
**Quartier Bergoue**
**F-64370 Arthez-de-Béarn (FR)**

**Goulas, Philippe**
**3 Place Mulot**
**F-64000 Pau (FR)**

(74) Mandataire: **Kohn, Armand**
**5 Avenue Foch**
**F-92380 Garches (FR)**

(54) **Procédé perfectionné de préparation de L-carnitine.**

(57) Procédé de préparation de la L-carnitine par réduction de la déhydrocarnitine au moyen de nicotinamide adénine dinucléotide (NADH), en présence de carnitine déshydrogénase (CDH), le NAD+ formé étant réduit par un formiate sous l'effet d'une formiate déshydrogénase (FDH) ; la solution aqueuse, constituant le milieu réactionnel, présente une force ionique d'au moins 0,5 M.

EP 0 240 423 A2

**Description**

Procédé perfectionné de préparation de L-carnitine.

L'invention concerne un procédé perfectionné pour la préparation de la carnitine, c'est-à-dire de l'acide triméthylamino-4 hydroxy-3 butyrique, et en particulier de la L-carnitine, par voie biochimique.

La carnitine est principalement employée pour des applications biologiques, notamment pharmacologiques, cosmétiques et alimentaires, en particulier comme stimulant de la sécrétion gastrique et pancréatique, ou comme antihyperlipoprotéinique. Comme les méthodes chimiques de sa préparation conduisent à des mélanges racémiques, alors que seul le stéréoisomère L est biologiquement actif, c'est surtout le procédé biochimique de préparation qui est intéressant, parce qu'il fournit l'isomère L. Il est donc important de disposer d'un procédé de préparation permettant l'obtention économique de ce composé. C'est l'avantage intéressant qu'apporte le procédé suivant l'invention ; il permet de produire, par voie biochimique, la L-carnitine avec bons rendements, pratiquement sans contamination par micro-organismes.

Un procédé connu de préparation de la L-carnitine consiste à réduire la déhydrocarnitine au moyen du nicotinamide adénine dinucléotide réduit, NADH, en présence de l'enzyme carnitine déshydrogénase (CDH) ; le NAD+ qui en résulte est réduit à son tour à l'état de NADH par un réducteur approprié, par exemple glucose, alcool, aldéhyde, dithionate de métal alcalin ou un formiate, en présence d'une déshydrogénase correspondante. Un tel procédé est notamment décrit dans le brevet français publié sous le No2 398 046.

En particulier la préparation de la L-carnitine est effec tuée par l'action du nicotinamide adénine dinucléotide réduit, NADH, sur la déhydrocarnitine, et réduction du NAD+ formé, au moyen du groupe formique avec de la formiate déshydrogénase FDH comme catalyseur.

Le schéma de synthèse est alors :

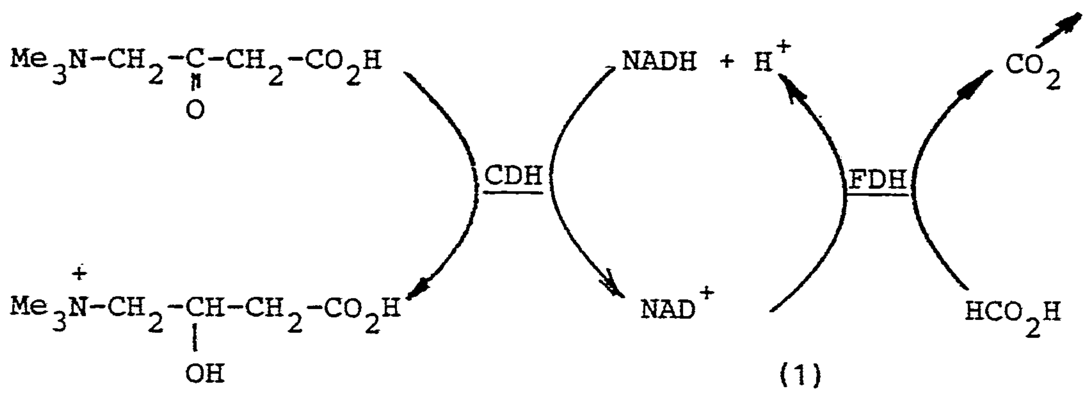


(1)

(L-carnitine) CDH = carnitine déshydrogénase

La CDH utilisée provient en général d'un extrait de culture bactérienne, par exemple de Pseudomona putida. Elle est précipitée au sulfate d'ammonium à 250 g par litre d'extrait brut (55% de la saturation).

Cependant la technique antérieure se prêtait mal à des applications industrielles, surtout à cause de très fortes quantités d'enzymes qu'elle exigeait par mole de L-carnitine à produire, et des productivités faibles par unité de capacité des réacteurs, par heure. L'utilisation de formiate comme réducteur du NAD+, tentante industriellement, a conduit jusqu'à présent à des rendements très bas, comme on peut le voir dans l'exemple 5 du brevet cité plus haut.

La présente invention résulte de la constatation que le procédé biochimique susindiqué peut être réalisé avec des quantités d'enzymes considérablement réduites, ramenées à des proportions industriellement acceptables, si le milieu réactionnel contient des sels dissous, dont la concentration dépasse un certain minimum.

La présence d'une telle force ionique exerce un effet sta bilisateur sur le système enzymatique présent, évitant sa contamination et destruction au cours de la réduction de la déhydrocarnitine.

Ainsi, le procédé suivant l'invention est-il caractérisé par le fait que le milieu aqueux, dans lequel a lieu la réaction (1), présente au cours de bioconversion une force ionique d'au moins 0,5 M et de préférence 0,6 à 3M, les forces ioniques de 0,8 à 2,5 M étant particulièrement favorables ; dans la technique antérieure citée, elles ne dépassaient pas 0,3 M.

Dans la forme préférée de l'invention, où une solution de déhydrocarnitine est versée progressivement dans une solution des enzymes requises, et que - par conséquent - le volume du milieu réactionnel varie du début à la fin de l'opération, la force ionique, variant en même temps, doit atteindre les limites susindiquées au moins à la fin de la réaction. Quant au début, il peut n'y avoir, dans la solution des enzymes, que 0,1 à 0,4 M de sels ionisables.

Malgré l'effet bénéfique, sur la stabilité des composants du milieu réactionnel, qu'exercent les électrolytes, il est préférable que la concentration de ceux-ci ne dépasse pas certaines limites, notamment la valeur d'environ 3M.

La force ionique adéquate peut être obtenue par la dissolution dans les liquides du milieu réactionnel de différents sels d'acides et bases organiques ou inorganiques. Le choix en est conditionné par le pH du milieu, qui doit être compris entre 6 et 8 et, de préférence, entre 6,8 et 7,8. A titre d'exemples non limitatifs de sels utilisables, on peut citer les chlorures, sulfates et phosphates de métaux alcalins, d'ammonium ou d'amines, ou bien des sels de tels cations avec différents acides organiques, comme formiates, tartrates, alkyl-sulfonates, aryl-sulfonates, phtalates, citrates, maléates, malonates ou autres.

L'introduction de l'électrolyte dans le milieu réactionnel peut avoir lieu de différentes manières : au départ, dans la solution des enzymes ; dans la solution de déhydrocarnitine que l'on verse progressivement dans la précédente; directement dans le milieu réactionnel, séparement de la solution de déhydrocarnitine. Deux de ces modes ou les trois à la fois peuvent être pratiqués conjointement. Comme il est commode d'employer la déhydrocarnitine sous la forme d'un sel, notamment de chlorhydrate, la réalisation de la force ionique appropriée peut s'opérer par l'introduction d'une solution de base, dans le milieu réactionnel, simultanément avec celle de la solution de déhydrocarnitine, ce qui conne lieu à la formation d'un sel, chlorure par exemple. Il est à noter à ce propos que la concentration en ions dans le milieu réactionnel dépend naturellement de celle du chlorhydrate ou autre sel de déhydrocarnitine dans la solution de cette dernière. Ainsi peut-on agir sur la force ionique par l'ajustement de cette concentration.

Le résultat remarquable du réglage adéquat de la force ionique, suivant l'invention, est d'abord la possibilité de diminuer considérablement la proportion d'enzymes ; ainsi, au lieu de 3200 à 8700 unités de carnitine déshydrogénase (CDH) utilisée par mole de déhydrocarnitine traitée, dans les exemples du brevet français cité plus haut, il suffit de 200 à 1000 unités, et le plus souvent de 400 à 900 U, donc environ 5 à 10 fois moins, ce qui constitue un progrès technique très important. En outre, grâce à la stabilité des enzymes assurée par la force ionique, on peut prolonger de beaucoup le temps de la réaction et arriver ainsi à des rendements beaucoup plus élevés que ne le permettrait la technique antérieure. La production horaire, par unité de volume de réacteur, est ainsi très fortement accrue.

Alors qu'il n'était guère possible de laisser la réaction se poursuivre plus de 40 heures, la présente invention permet de dépasser largement 48 heures et atteindre par exemple une centaine d'heures. On peut en général travailler durant 45 à 80 heures.

Un autre résultat intéressant est que les enzymes employées conservent encore la majeure partie de leur activité initiale, après une préparation de L-carnitine ; elles peuvent ainsi être réutilisées pour une nouvelle opération.

De préférence, la température ne dépasse pas 30°C ; elle peut varier entre 10° et 30°C.

Les enzymes, employées dans le procédé de l'invention, sont bien connues dans l'art, il n'y a donc pas lieu de décrire ici leur préparation. On notera seulement que la carnitine déshydrogénase (CDH) peut être obtenue à partir de l'extrait des cultures bactériennes de Pseudomona des groupes fluorescens, seruginosa et surtout putida (collection de l'Institut Pasteur no CIP 52191).

La formiate déshydrogénase (FDH) peut être obtenue à partir de divers micro-organismes, de la façon connue, et en particulier à partir des levures des genres Candida, Kloeckera, Pichia, Torulopsis etc. et surtout à partir de Candida bodiini.

Etant donné la fragilité des solutions aqueuses de déhydrocarnitine (DHC) aux températures supérieures à 10°C, il n'est pas recommandable de mettre d'emblée la totalité de DHC dans le réacteur contenent une solution d'enzyme CDH.

Le mode opératoire préféré consiste à conserver la solution de DHC dans un réservoir à part, à température suffi samment basse, par exemple entre 0° et 4°C, et n'en introduire progressivement, dans le réacteur, qu'un certain courant compatible avec la vitesse de réaction, de façon à ce que la DHC soit pratiquement convertie en L-carnitine au fur et à mesure de son arrivée dans le réacteur.

Cela conduit à la forme préferée du procédé de l'invention suivante. Dans un réacteur, muni de moyens d'agitation et de réglage de la température, on introduit un volume déterminé d'une solution de NADH et des deux enzymes, CDH et FDH, convenablement tamponnée à un pH voisin de 7.

On y ajoute également, selon l'invention, un sel de manière à ce que la force ionique de la solution d'enzymes soit d'au moins 0,2 M.

A cette solution maintenue entre 10° et 30°C, en particulier à 20°C, on ajoute progressivement, sous agitation, une solution aqueuse de déhydrocarnitine, venant d'un réservoir spécial où cette solution de DHC est stockée à basse température. Connaissant, au moins approximativement, la vitesse de la réaction :

(2) $DHC + NADH \xrightarrow{CDH} L\text{-carnitine} + NAD$

on règle le débit de la solution de telle manière que la DHC soit, au moins en majeure partie, convertie au fur et à mesure de son arrivée dans la solution .

De préférence, la DHC étant sous la forme de son chlorhydrate DHC.HCl, soit

$$\overset{-}{Cl},Me_3\overset{+}{N}-CH_2-\underset{\overset{\|}{O}}{C}-CH_2COOH$$

on fait couler simultanément, dans le réacteur, un débit de solution basique, juste pour neutraliser l'acidité de la DHC introduite ; la solution basique peut être par exemple de NaOH, KOH, $CH_3COONa$ etc. et, plus

particulièrement de l'ammoniaque qui donne du $NH_4Cl$ bien favorable à la force ionique du milieu.

La concentration en DHC de la solution aqueuse, utilisée, peut varier entre les larges limites, mais elle est le plus souvent de l'ordre de 0,2 à 2 M ; une des incidences de ce facteur est expliquée plus haut au sujet du réglage de la force ionique.

Lorsque la totalité de DHC a été introduite dans le réacteur, on détermine la teneur du milieu en la L-carnitine formée ; on procéde ensuite à la séparation et la purification de celle-ci selon la technique connue.

L'invention est illutrée par les exemples non limitatifs qui suivent.

EXEMPLE 1

Dans un réacteur de 250 ml de capacité on a placé 50 ml d'une solution aqueuse renfermant les composants suivants :

40 U d'enzyme carnitine déshydrogénase CDH présentant une activité spécifique de 55U/mg provenant du Pseudomona putida ;
2mM de NAD+ ;
40 U formiate déshydrogénase, FDH, provenant de la levure Candida bodiini (EC 1.2.1.2. ; vendue par BOEHRINGER MANNHEIM)
50 mM de phosphates $HNa_2PO_4/KH_2PO_4$ (pH 7,5)
150mM de formiate d'ammonium $HCOONH_4$
6 mg de chloramphénicol

Dans cette solution de départ on a fait couler, sous agitation, à raison de 1,2 ml/h, une solution aqueuse
0,8 M de chlorhydrate de DHC et
0,8 M d'acide formique
provenant d'un réservoir dans lequel cette solution était stockée à 4°C.

La température dans le réacteur était réglée à 30°C. Simultanément avec l'introduction de la solution de DHC, on ajustait le pH du milieu réactionnel à la valeur de 7, par l'injection d'un solution 2N de $NH_4OH$.

Après 70 heures, le volume de solution dans le réacteur est de 150 ml et sa concentration en L-carnitine 428 mM.

Le bilan de l'opération se présente donc comme suit :
0,0672 mole de DHC mis en oeuvre,
0,0642 mole de L-carnitine formée (10,3g) soit rendement de 96 %
Ce résultat est obtenue avec :
force ionique au départ 0,20M
force ionique à la fin 0,96M
nombre d'unités CDH par mole de DHC 595

EXEMPLE 2

Cherchant à limiter l'augmentation de volume au cours de la bioconversion, on a mis en oeuvre des réactifs en solutions plus concentrées en déhydrocarnitine, acide formique et ammoniaque. D'autre part, on a réduit la concentration en NAD+.

Ainsi a-t-on chargé dans le réacteur 50 ml de solution contenant :
$HNa_2PO_4/KH_2PO_4$ 50 mM pH : 7,5
NAD+ 0,2 mM $HCOONH_4$ 150 mM
6 mg chloramphénicol
40 U de CDH sommairement purifiée comme décrit ci-après
25U de FDH BOEHRINGER-MANNHEIM provenant de la Candida bodiini.

La CDH, extraite de P. Putida, avait été précipitée au sulfate d'ammonium (55 % de la saturation), centrifugée et le culot redissous dans le tampon phosphate 50mM pH : 7,5 ; la solution obtenue dialysée pendant 16 h contre ce même tampon.

Le réacteur était alimenté avec une solution maintenue à 4°C, contenant la déhydrocarnitine et l'acide formique à la concentration, l'un et l'autre, de 1,6 M. Le débit était de 0,6 ml/h. La température du réacteur maintenue à 30°C et le pH 7,5 par addition contrôlée d'ammoniaque 8N. Après 48 heures, on atteint un volume final de 90 ml et une concentration en L-Carnitine de 500 mM, soit un rendement de 98 %.

La force ionique au début était de ............0,20
La force ionique à la fin était de ............1,13
Unités CDH/mole DHC ..........................868

EXEMPLE 3

La synthèse de L-carnitine a été effectuée avec, au départ, 1 litre de solution , dans les conditions initiales suivantes :
$Na_2HPO_4/KH_2PO_4$ 50 mM pH : 7,5
$HCO_2NH_4$ 150 mM
NAD+ 0,6 mM
120 mg de chloramphénicol
CDH 800 U sommairement purifiée comme dans l'exemple 2
FDH 500 U soit 750 mg du lyophilisat commercialisé par Boehringer-Mannheim.

Le réacteur était alimenté par la même solution 0,8 M que dans l'exemple 1, avec un débit de 0,4 ml/min. Le pH était maintenu à 7,5, par un système régulateur adapté au réacteur, avec de l'ammoniaque 2 N ; la température était fixée à 30°C. Une agitation mécanique assurait l'homogénéisation de l'ensemble.

Après 67 h on a atteint un volume de 3,2 l et une concentration en L-carnitine de 390 mM soit un rendement de 97 %.

Dans cet exemple on a travaillé avec :

une force ionique initiale de.......... 0,20

une force ionique finale de ........... 0,86

un rapport unités CDH/moles DHC........ 622

Le dosage de la CDH présente dans le réacteur donne 704 U soit 88 % d'activité résiduelle. Pour la FDH le dosage donne 384 U soit 80 % d'activité résiduelle. Le NAD+ a été intégralement transformé en NADH par voie enzymatique,puis dosé spectrophotométriquement à 340 mm. On obtient 0,2 mM ce qui , compte tenue de la dilution, correspond à 100 % d'activité.

EXEMPLE 4

En partant d'un litre de solution,on a effectué la synthèse de la L-carnitine avec les conditions initiales suivantes :

tampon phosphate 50 mM pH 7,5

$HCO_2NH_4$ 0,5 M

NAD+ 0,4 mM

CDH 820 U purifiée comme dans l'exemple 2

FDH 503 U extraite d'une culture de Pichia Pastoris, précipitée au sulfate d'ammonium (50 % de la saturation) , centrifugée et le culot redissous dans du tampon phosphate 50 mM pH 7,5 , la solution obtenue dialysée 16 h contre ce même tampon.

Le réacteur était alimenté par la même solution de DHC que dans l'exemple 2, avec un débit de 12 ml/h.

Après 49 h on atteint un volume de 1,75 litres et une concentration en L-carnitine de 526 mM, soit un rendement de 98 %. Le dosage des activités résiduelles en CDH et FDH a donné respectivement 459 U et 111 U.

Au cours de cette préparation la force ionique du milieu réactionnel était :

au départ 0,505

à la fin 1,363

et le rapport CDH/mol DHC = 872 unités/mol La production ressort à 1,72 g de L-carnitine par heure, par litre de volume liquide final.

La force ionique , imposée par les sels (formiate, phosphate, chlorure d'ammonium) s'est révelée suffisante pour retarder toute contamination bactérienne et permettre le travail sans antibactériens tels que le chloramphénicol.

EXEMPLE 5

Dans le but d'atteindre une concentration finale en L-carnitine plus élevée, un réacteur a été initialement chargé de 50 ml de solution :

tampon phosphate 50 mM pH 7,5

NAD+ 0,4 mM

$HCO_2NH_4$ 0,5 M

CDH : 80 U purifiée comme à l'exemple 2

FDH : 50 U purifiée comme à l'exemple 4

Le réacteur était alimenté par une solution aqueuse de déhydrocarnitine DHC 3,2 M, mélangée à de l'acide formique 3,2 M, avec un débit de 0,55 ml/h, le pH dans le réacteur étant maintenu à 7,5 au moyen d'ammoniaque.

Après 47 h on atteint un volume final de 82 ml et une concentration en L-carnitine de 880 mM soit un rendement de 87 %. Les activités en CDH et FDH étaient respectivement de 21 U et 10 U.

Les forces ioniques étaient dans cet essai : au départ 0,505 M, à la fin 2,3 M.

Rapport unités CDH/moles DHC = 967 et la production 3 g/h par litre de volume final ; on peut constater que cette productivité augmentée a été obtenue au prix d'une légère baisse du rendement.

Comme dans l'exemple 4 l'absence d'antibactérien est justifiée par la force ionique encore plus élevée du milieu, ce qui retarde toute contamination bactérienne.

EXEMPLE 6

On opère de la même façon que dans l'exemple 3, mais la solution de mélange de DHC + acide formique n'est que 0,1 M. La force ionique du milieu passe de 0,20 M de début à 0,16 M après 67 heures de réaction, car la dilution compense largement l'ajout de sels en cours de la bioconversion.

Le rendement en L-carnitine n'est plus que de 60%, et les activités résiduelles en enzymes sont nulles du fait d'une contamination du milieu. La production est de 0,07 g/h, par litre de volume final.

EXEMPLE 7

La réaction est mise en route comme dans l'exemple 1, mais avec une concentration initiale de formiate de 3,0 M au lieu de 0,15 M. La solution de DHC ne contient alors pas de HCOOH.

La force ionique de 3,05 M au départ passe à 3,45 M à la fin. Le rendement est de 40%. Aucune contamination ne s'est produite, mais l'activité de la CDH baisse à 15% de sa valeur initiale. La production est de 1,03 g/h x l de volume final.

Récapitulation des exemples.

Dans le tableau ci-après on a reporté les principaux résultats des exemples qui précèdent. Les abréviations suivantes sont adoptées :

F.i.i ..... force ionique initiale, en moles par litre

F.i.f ..... force ionique finale,en M.

CDH/DHC.... nombre d'unités CDH employées par mole de DHC.

Rdt. % rendement en L-carnitine sur la DHC consommée.

Prod. ... g de L-carnitine obtenue par heure, par litre de solution finale.

| Exemple n° | F.i.i | F.i.f | CDH/DHC | Rdt.% | Prod. |
|---|---|---|---|---|---|
| 1 | 0,20 | 0,96 | 595 | 96 | 0,98 |
| 2 | 0,20 | 1,13 | 868 | 98 | 1,67 |
| 3 | 0,20 | 0,86 | 622 | 97 | 0,93 |
| 4 | 0,50 | 1,36 | 872 | 98 | 1,72 |
| 5 | 0,50 | 2,30 | 967 | 87 | 3,0 |
| 6 | 0,20 | 0,16 | 4975 | 60 | 0,07 |
| 7 | 3,05 | 3,45 | 595 | 40 | 1,03 |

Les exemples 1 à 5 représentent visiblement des cas optimaux, alors que les exemples 6 et 7 montrent une chute du rendement lorsque la force ionique est trop faible (ex.6) ou trop forte (ex.7).

D'autre part, l'exemple 5 indique une certaine limite à la vitesse de l'opération : une production assez forte (3g/l.h.) est obtenue au prix d'une baisse de rendement (87 %).

**Revendications**

1. Procédé de préparation de la L-carnitine par réduction de la déhydrocarnitine au moyen de nicotinamide adénine dinucléotide (NADH), en présence de carnitine déshydrogénase (CDH), le NAD+ formé étant réduit par un formiate sous l'effet d'une formiate déshydrogénase (FDH), caractérisé en ce que la solution aqueuse, constituant le milieu réactionnel, présente, au cours de bioconversion, une force ionique d'au moins 0.5 M.

2. Procédé suivant la revendication 1, caractérisé en ce que la force ionique du milieu réactionnel est de 0,6 à 3 M, au moins à la fin de l'opération.

3. Procédé suivant la revendication 1, dans lequel une solution aqueuse de déhydrocarnitine est versée progressivement dans une solution des enzymes, caractérisé en ce que le milieu réactionnel présente une force ionique de 0,1 à 0,4 M au départ et 0,8 à 2,5 M à la fin de l'opération.

4. Procédé suivant une des revendications 1 à 3, caractérisé en ce que la force ionique est formée principalement par du formiate d'ammonium.

5. Procédé suivant une des revendications 1 à 4, dans lequel la déhydrocarnitine est prise sous la forme de chlorhydrate, le milieu réactionnel étant neutralisé avec de l'ammoniaque, caractérisé en ce qu'une solution d'acide formique est versé dans ce milieu en même temps que la solution de déhydrocarnitine.

6. Procédé suivant une des revendications précédentes, caractérisé en ce que le milieu réactionnel contient 200 à 1000 unités d'enzyme CDH par mole de déhydrocarnitine employée au total.

7. Procédé suivant une des revendications précédentes, caractérisé en ce que la solution de déhydrocarnitine a une concentration de 0,2 à 2 M.

8. Procédé suivant la revendication 7, caractérisé en ce que cette solution est versée dans la solution des enzymes avec une vitesse telle que la déhydrocarnitine soit convertie en L-carnitine au fur et à mesure de son arrivée dans la solution enzymatique.